# EUROPÄISCHE PATENTSCHRIFT

(11) **EP 1 149 084 B1**
(45) Veröffentlichungstag und Bekanntmachung des Hinweises auf die Patenterteilung: **10.09.2003**
(21) Anmeldenummer: 00903599.9
(22) Anmeldetag: 21.01.2000
(51) Int. Cl.: C07D 307/62

(54) **VERFAHREN ZUR HERSTELLUNG VON L-ASCORBINSÄURE**
METHOD FOR PRODUCING L-ASCORBIC ACID
PROCEDE DE PREPARATION D'ACIDE L-ASCORBIQUE

(30) Priorität: 05.02.1999 DE 19904821
(43) Veröffentlichungstag der Anmeldung: 31.10.2001
(73) Patentinhaber: MERCK PATENT GmbH, 64293 Darmstadt (DE)
(72) Erfinder: FECHTEL, Ulrich, D-64372 Ober-Ramstadt (DE); HEINZ, Wolfgang, D-62625 Bensheim (DE); MÜLLER, Bernd, D-64673 Zwingenberg (DE); BESCHMANN, Klaus, D-64354 Reinheim (DE); STOLDT, Jöran, D-64331 Weiterstadt (DE)
(86) Internationale Anmeldenummer: EP0000442
(87) Internationale Veröffentlichungsnummer: WO00046216

(56) Entgegenhaltungen:
- WO-A-87/00839
- DE-C- 19 734 086
- GB-A- 2 034 315
- GB-A- 2 205 567

## Beschreibung

Die Erfindung betrifft ein Verfahren zur Herstellung von L-Ascorbinsäure aus 2-Keto-L-gulonsäure oder 2,3-4,6-Diaceton-2-keto-L-gulonsäure, wobei die Reaktion in Gegenwart von Wasser und Halogenwasserstoff durchgeführt wird und die Konzentration des Halogenwasserstoffs in Wasser größer als 37 Gew.% ist.

Herstellverfahren für L-Ascorbinsäure basierend auf der Umsetzung von 2-Keto-L-gulonsäure oder 2,3-4,6-Diaceton-2-keto-L-gulonsäure sind bereits bekannt. Bei Einsatz der 2-Keto-L-gulonsäure sind in der Literatur sowohl das Esterverfahren über die Stufen 2-Keto-Lgulonsäuremethylester und Natriumascorbat als auch direkte Verfahren mit Säuren beschrieben. Bei den direkten Verfahren wird nach Enolisierung und Lactonisierung von 2-Keto-L-gulonsäure L-Ascorbinsäure erhalten. In der direkten Umwandlung wird in den bekannten Verfahren vorzugsweise Salzsäure als Katalysator verwendet. Meistens wird dabei in Gegenwart von organischen Lösungsmitteln wie Toluen, Xylen, Aceton, Chloroform usw. gearbeitet. Nachteile dieser bekannten Verfahrensweise sind jedoch zum Beispiel die langen Reaktionszeiten und die Notwendigkeit des Einsatzes und der Aufarbeitung von Lösungsmittelgemischen.

Die Umsetzung der 2-Keto-L-gulonsäure mit 36%iger Salzsäure ist z.B. in der DE 29 39 052 beschrieben. Nach einer Reaktion bei 100 °C und nach Abdestillation der Salzsäure wird eine Ausbeute von 87 % der Theorie an L-Ascorbinsäure erhalten. Der Nachteil des Verfahrens ist allerdings die schnelle Zersetzung der Ascorbinsäure bei 100°C, so daß es zu einer vermehrten Bildung von Nebenprodukten und zu einer intensiven Schwarzfärbung der Lösung kommt. Aufgrund der großen Menge an Nebenprodukten ist eine Isolierung der Ascorbinsäure mit weiteren, nicht unerheblichen Materialverlusten verbunden.

Die oben genannten Probleme wurden entsprechend dem in der Patentschrift DE 197 34 086 vorgeschlagenen Verfahren teilweise behoben. Durch Absenkung der Umsetzungstemperatur auf 40 bis 80 °C können bei gleichzeitiger Verlängerung der Reaktionszeit und in Gegenwart von 37%iger Salzsäure höhere Ausbeuten an Ascorbinsäure in Lösung erhalten werden. Beispielsweise kann bei einer Umsetzungstemperatur von 58 °C bis zu 91 % an Ascorbinsäure in Lösung erhalten werden. Diese Lösung enthält jedoch wasserunlösliche schmierige Nebenprodukte und ist schwarz gefärbt, so daß vor einer Kristallisation der Ascorbinsäure die Nebenprodukte und insbesondere die unerwünschte schwarze Farbe entweder durch Aktivkohlebehandlung oder aber durch Extraktion bzw. Waschung mit einem organischen Lösungsmittel entfernt werden muß. Zudem muß die nach Kristallisation erhaltene Roh-Ascorbinsäure aus Qualitätsgründen nochmals einem Entfärbungsschritt, beispielsweise einer weiteren Aktivkohlebehandlung, und einer zusätzlichen Kristallisation unterzogen werden.

Eine Verbesserung dieser Ergebnisse konnte durch eine weitere Absenkung der Reaktionstemperaturen bei der Anwendung des in der DE 197 34 086 beschriebenen Verfahrens nicht erzielt werden. Eine Reaktionstemperatur unter 50°C verlangsamt beispielsweise die Umsetzungsgeschwindigkeit in der Weise, daß die Reaktionszeiten signifikant ansteigen. Zudem können die farbgebenden Zersetzungsreaktionen bei dieser Reaktionstemperatur nicht zurückgedrängt werden. Auch bei noch tieferen Reaktionstemperaturen von 40 °C werden z.B. bei einem unvollständigen Umsatz der 2-Keto-L-gulonsäure (77 % Ausbeute) wasserunlösliche und intensiv schwarz gefärbte Nebenprodukte gebildet. Die Aufarbeitung erfordert deshalb die vorherige Abtrennung dieser Nebenprodukte.

Auch in der GB 22 05 567 A wird die Umwandlung von 2-Keto-L-Gulonsäure in L-Ascorbinsäure in Gegenwart von organischen Lösungsmitteln und einem oberflächenaktiven Mittel bewirkt, hier in Gegenwart von Wassser, einem inerten organischen Lösungsmittel und einem aliphatischen Keton. Dabei wird Salzsäure mit einer Konzentration von 20 bis 45 % eingesetzt, deren Konzentration in der Reaktionsmischung allerdings durch mit anderen Komponenten eingetragenes Wasser deutlich reduziert wird. Die Reaktion findet bei Atmosphärendruck statt.

Die in dem Verfahren eingesetzten organischen Medien verteuern und komplizieren sowohl die Durchführung der Reaktion als auch die Aufarbeitung des Reaktionsgemisches.

Somit bestand die Aufgabe, ein Verfahren zur Herstellung von L-Ascorbinsäure aus 2-Keto-L-gulonsäure oder 2,3-4,6-Diaceton-2-keto-L-gulonsäure zu entwickeln, welches die Nachteile der bekannten Verfahren vermeidet oder zumindest vermindert. Insbesondere soll durch dieses Verfahren eine hohe Ausbeute an L-Ascorbinsäure ermöglicht werden und außerdem L-Ascorbinsäure in einer solchen Qualität anfallen, daß der Aufwand zur Entfärbung der Reaktionslösung möglichst gering gehalten werden kann.

Überraschenderweise wurde nun gefunden, daß diese Aufgabe gelöst wird, wenn das Verfahren zur Herstellung von L-Ascorbinsäure aus 2-Keto-L-gulonsäure oder 2,3-4,6-Diaceton-2-keto-L-gulonsäure so durchgeführt wird, daß die Reaktion in Gegenwart von nur Wasser und Halogenwasserstoff stattfindet und die Konzentration des Halogenwasserstoffs in Wasser nach Zugabe aller Reaktionskomponenten von 40 bis 90 Gew. % und der Druck 10 bis 100 bar beträgt.

Das erfindungsgemäße Verfahren ermöglicht eine sehr gute Ausbeute an L-Ascorbinsäure. Die nach dem erfindungsgemäßen Verfahren hergestellte L-Ascorbinsäure fällt außerdem in einer solchen Qualität an, daß der Aufwand zur Entfärbung der Reaktionslösung sehr gering ist. Zudem kann die gestellte Aufgabe trotz niedriger Reaktionstemperaturen mit kurzen Reaktionszeiten gelöst werden.

Für das erfindungsgemäße Verfahren wird vorzugsweise 2-Keto-L-gulonsäure als Edukt verwendet.

Für das erfindungsgemäße Verfahren sind die Halogenwasserstoffe HF, HCl, HBr und HI geeignet. Bevorzugt wird HCI oder HBr für das erfindungsgemäße Verfahren verwendet. Insbesondere bevorzugt wird HCI für das erfindungsgemäße Verfahren verwendet.

Aus der Literatur bekannte Sättigungskonzentrationen von Halogenwasserstoffen in Wasser unter Atmosphärendruck sind beispielsweise für HCl 45 Gew.% bei 0 °C, 42,7 Gew.% bei 25 °C, 40,2 Gew.% bei 30 °C, 38,9 Gew.% bei 40 °C, 37,3 Gew.% bei 50 °C und 35,9 Gew.% bei 60 °C, für HBr 68,9 Gew.% bei 0 °C und 66 Gew.% bei 25 °C und für HI 90 Gew.% bei 0 °C und 70 Gew.% bei 10°C. Die Sättigungskonzentration der Halogenwasserstoffe in Wasser kann nach bekannten Methoden bestimmt werden.

Das erfindungsgemäße Verfahren kann beispielsweise derart ausgeführt werden, daß 2-Keto-L-Gulonsäure oder 2,3-4,6-Diaceton-2-keto-Lgulonsäure und Halogenwasserstoff in einem Autoklav vorgelegt werden, wobei der Halogenwasserstoff üblicherweise in handelsüblicher Form verwendet wird (HCI z.B. in Form einer 37 Gew.%igen wäßrigen Lösung, die im Rahmen der vorliegenden Erfindung auch als konz. Salzsäure bezeichnet wird). Anschließend wird gasförmiger unverdünnter Halogenwasserstoff solange zugegeben bzw. eingeleitet, bis die gewünschte Konzentration des Halogenwasserstoffs in Wasser erreicht worden ist.

Alternativ können z.B. auch 2-Keto-L-gulonsäure oder 2,3-4,6-Diaceton-2-keto-L-gulonsäure und Wasser in einem Autoklav vorgelegt werden und nach Verschließen des Autoklaven eine derartige Menge an unverdünntem Halogenwasserstoff in flüssiger Form zugegeben bzw. eingeleitet werden, daß die gewünschte Konzentration des Halogenwasserstoffs in Wasser erreicht wird.

Unverdünnter Halogenwasserstoff bedeutet im Rahmen der vorliegenden Erfindung insbesondere, daß der Halogenwasserstoff kein oder nur wenig Wasser enthält.

Zur Durchführung der Reaktion wird das Reaktionsgemisch nach der Zugabe bzw. dem Einleiten des gasförmigen oder flüssigen unverdünnten Halogenwasserstoffs auf Reaktionstemperatur gebracht, gegebenenfalls durch Erwärmen, und für eine bestimmte Zeitdauer bei dieser Temperatur belassen.

Für das erfindungsgemäße Verfahren sind Reaktionstemperaturen von 0 bis 60 °C geeignet. Vorzugsweise arbeitet man bei Temperaturen von 25 bis 50 °C und besonders bevorzugt bei Temperaturen von 35 bis 45 °C.

Bei Verwendung der Halogenwasserstoffe HF, HBr und HI wird das erfindungsgemäße Verfahren vorzugsweise unter Atmosphärendruck durchgeführt. Bei Verwendung des Halogenwasserstoffs HCI wird das erfindungsgemäße Verfahren jedoch bei einem Druck durchgeführt, welcher im Vergleich zu Atmosphärendruck erhöht ist. Dieser Druck beträgt besonders bevorzugt von 10 bis 100 bar und insbesondere bevorzugt von 10 bis 50 bar. Der Druck kann bei Durchführung des erfindungsgemäßen Verfahrens bis zu 150 bar betragen.

Die Reaktion kann sowohl diskontinuierlich als auch kontinuierlich erfolgen. Eine kontinuierliche Verfahrensweise ist jedoch bei vollständiger Auflösung der 2-Keto-L-gulonsäure bevorzugt, da hierdurch Zeitregimes besser eingehalten werden können. Die kontinuierliche Verfahrensweise findet vorzugsweise in einem druckfesten Strömungsrohr statt. Das Verfahren kann zudem wesentlich vereinfacht werden, wenn der für die Reaktion benötigte Halogenwasserstoff durch Destillation und Kompression wieder recycelt wird. Bei dieser bevorzugten Ausführungsform des Verfahrens ist der Bedarf an Halogenwasserstoff gering.

Im folgenden wird das erfindungsgemäße Verfahren am Beispiel der 2-Keto-L-gulonsäure und des besonders bevorzugten Halogenwasserstoffs HCI näher beschrieben. Für 2,3-4,6-Diaceton-2-keto-Lgulonsäure und die anderen Halogenwasserstoffe kann das erfindungsgemäße Verfahren jedoch analog durchgeführt werden.

Der Anteil der 2-Keto-L-gulonsäure in der Einsatzmischung vor dem Eintrag des Chlorwasserstoffs kann von 15 bis 97 Gew.% bezogen auf die gesamte Einsatzmischung vor dem Eintrag des Chlorwasserstoffs betragen. Vorzugsweise beträgt der Anteil der 2-Keto-L-gulonsäure vor dem Eintrag des Chlorwasserstoffs von 30 bis 40 Gew.% bezogen auf die gesamte Einsatzmischung vor dem Eintrag des Chlorwasserstoffs.

Vorzugsweise wird Chlorwasserstoff bei Temperaturen von 0 bis 60 °C, besonders bevorzugt bei Temperaturen von 0 bis 30 °C und insbesondere bevorzugt bei etwa 15 bis 25 °C eingeleitet, die Reaktionsmischung auf die gewünschte Reaktionstemperatur gebracht, gegebenenfalls durch Erwärmen, und für eine bestimmte Zeitdauer bei dieser Temperatur belassen.

Das Verfahren kann so gestaltet werden, daß die 2-Keto-L-gulonsäure vor dem Eintrag des Chlorwasserstoffs als Feststoff oder als Lösung in Wasser oder wäßriger HCI vorliegt. Je nach der Menge des vorgelegten Wassers bzw. der vorgelegten wäßrigen HCI lassen sich HCI-Konzentrationen bis nahezu 100 Gew.% HCI in Wasser realisieren.

Jedoch auch bei Einsatz der 2-Keto-L-gulonsäure als Feststoff ist ein geringfügiger Wasseranteil sinnvoll, vorzugsweise mindestens 3 Gew.% Wasser bezogen auf die eingesetzte 2-Keto-L-gulonsäure, da für die Umsetzung Wasserspuren essentiell sind. Je geringer die Wasseranteile in der Reaktionsmischung sind, desto langsamer verläuft die Reaktion. Offensichtlich reagiert die 2-Keto-L-gulonsäure lediglich im gelösten Zustand. Unter Berücksichtigung dieses Befunds können die Wassermengen ansonsten über weite Konzentrationsbereiche variiert werden.

Nach Zugabe aller Reaktionskomponenten beträgt die Konzentration von Chlorwasserstoff in Wasser vorzugsweise von 40 bis 90 Gew.%, besonders bevorzugt von 42,7 bis 90 Gew.% und insbesondere bevorzugt von 45 bis 65 Gew.%.

Die Halogenwasserstoffe HF, HBr und Hl können z.B. in käuflich erwerbbaren Konzentrationen in Wasser für das erfindungsgemäße Verfahren verwendet werden, z.B. HBr und HI in Konzentrationen von etwa 47 bis 48 Gew.% in Wasser und HF in Konzentrationen von etwa 45 bis 70 Gew.% in Wasser. Falls höhere Konzentrationen als die soeben genannten Konzentrationen für die Durchführung des erfindungsgemäßen Verfahrens mit HF, HBr und HI erwünscht sind, kann die gewünschte Konzentration des Halogenwasserstoffs in Wasser durch Zugabe von gasförmigem oder flüssigem unverdünntem Halogenwasserstoff eingestellt werden. Im Fall des Halogenwasserstoffs HCI wird die gewünschte Konzentration an HCI in Wasser vorzugsweise durch Zugabe von gasförmigem oder flüssigem unverdünntem Halogenwasserstoff eingestellt.

Nach Zugabe aller Reaktionskomponenten beträgt das Gewichtsverhältnis von Chlorwasserstoff zu 2-Keto-L-gulonsäure vorzugsweise von 0,5 : 1 bis 8 : 1 und besonders bevorzugt von 2 : 1 bis 4 : 1.

Nach Zugabe aller Reaktionskomponenten beträgt das Gewichtsverhältnis von Wasser zu 2-Keto-L-gulonsäure vorzugsweise von 3 : 100 bis 100 : 10 und besonders bevorzugt von 1 : 2 bis 3 : 1.

Das erfindungsgemäße Verfahren kann auch bei überkritischen Bedingungen durchgeführt werden. Beispielsweise werden für das HCI-Gas bei 51 °C und 80 bar überkritische Bedingungen erreicht.

Üblicherweise kann die Reaktion bei Verwendung aller Halogenwasserstoffe nach etwa 1 bis 4 Stunden durch Druckentspannung und eventuelles Abkühlen des Reaktionsgemischs abgebrochen werden. Das Fortschreiten bzw. das Ende der Reaktion kann auch nach geeigneter Probenentnahme und Analyse der Probe mittels HPLC oder lodometrie überprüft werden.

Die Aufarbeitung der Ansätze kann für alle Halogenwasserstoffe nach der Druckentspannung durch Entfernung des Halogenwasserstoffs, beispielsweise durch Destillation, erfolgen. Der resultierende Feststoff kann gegebenenfalls mit einem organischen Lösungsmittel wie z.B. einem Alkohol, vorzugsweise Butanol, angeschlämmt werden, so daß sowohl die geringfügig vorhandenen farbgebenden Substanzen als auch Reste des Halogenwasserstoffs bei einer anschließenden Destillation entfernt werden können.

Nachfolgend sind Beispiele aufgeführt, die die Erfindung erläutern sollen ohne sie zu begrenzen.

Nach der Isolierung der L-Ascorbinsäure wurde die Ausbeute entweder in Lösung oder im Feststoff mittels lodometrie der L-Ascorbinsäure ermittelt.

### Beispiele

Verfahrensvariante A:

### Beispiel 1

3 g 2-Keto-L-gulonsäure werden in einem Glaseinsatz vorgelegt und anschließend werden 9 g konz. Salzsäure hinzuaddiert. Nach Verschließen des Autoklaven wird Chlorwasserstoff so lange eingeleitet bis kein Druckabfall durch die Auflösung der HCI in der wäßrigen Phase mehr auftritt (ca. 20 min.). Der Autoklav wird anschließend innerhalb von 0,5 h auf 30 °C aufgewärmt und bei dieser Temperatur drei Stunden gehalten (Druck ca. 43 bar). Nach dem Abkühlen und Entspannen des Autoklaven wird die Reaktionsmischung am Rotationsverdampfer eingeengt. Gegen Ende fällt ein fast weißes kristallines Material an, welches zur Entfernung von Säurespuren nochmals in Butanol aufgenommen wird und anschließend wiederum zum Rückstand gebracht wird (Ausbeute: 82 % der Theorie).

### Beispiel 2

3 g 2-Keto-L-gulonsäure werden in einem Glaseinsatz vorgelegt und anschließend werden 9 g konz. Salzsäure hinzuaddiert. Nach Verschließen des Autoklaven wird Chlorwasserstoff so lange eingeleitet bis kein Druckabfall durch die Auflösung der HCI in der wäßrigen Phase mehr auftritt (ca. 20 min.). Der Autoklav wird anschließend innerhalb von 0,5 h auf 40 °C aufgewärmt und bei dieser Temperatur drei Stunden gehalten (Druck ca. 45 bar). Nach dem Abkühlen und Entspannen des Autoklaven wird die Reaktionsmischung am Rotationsverdampfer eingeengt. Gegen Ende fällt ein hellgraues kristallines Material an, welches zur Entfernung von Säurespuren nochmals in Butanol aufgenommen wird und anschließend wiederum zum Rückstand gebracht wird (Ausbeute: 87,1 % der Theorie).

### Beispiel 3

2 g 2-Keto-L-gulonsäure werden in einem Glaseinsatz vorgelegt und anschließend werden 9 g konz. Salzsäure hinzuaddiert. Nach Verschließen des Autoklaven wird Chlorwasserstoff so lange eingeleitet bis kein Druckabfall durch die Auflösung der HCI in der wäßrigen Phase mehr auftritt (ca. 20 min). Der Autoklav wird anschließend innerhalb von 0,5 h auf 42 °C aufgewärmt und bei dieser Temperatur drei Stunden gehalten (Druck ca. 45 bar). Nach dem Abkühlen und Entspannen des Autoklaven wird die Reaktionsmischung am Rotationsverdampfer eingeengt. Gegen Ende fällt ein hellgraues kristallines Material an, welches zur Entfernung von Säurespuren nochmals in Butanol aufgenommen wird und anschließend wiederum zum Rückstand gebracht wird (Ausbeute: 95 % der Theorie).

### Beispiel 4

3 g 2-Keto-L-gulonsäure werden in einem Glaseinsatz vorgelegt und anschließend werden 9 g konz. Salzsäure hinzuaddiert. Nach Verschließen des Autoklaven wird Chlorwasserstoff so lange eingeleitet bis kein Druckabfall durch die Auflösung der HCl in der wäßrigen Phase mehr auftritt (ca. 20 min). Der Autoklav wird anschließend innerhalb von 0,5 h auf 42 °C aufgewärmt und bei dieser Temperatur drei Stunden gehalten (Druck ca. 45 bar). Nach dem Abkühlen und Entspannen des Autoklaven wird die Reaktionsmischung am Rotationsverdampfer eingeengt. Gegen Ende fällt ein hellgraues kristallines Material an, welches zur Entfernung von Säurespuren nochmals in Butanol aufgenommen wird und anschließend wiederum zum Rückstand gebracht wird (Ausbeute: 93,3 % der Theorie).

### Beispiel 5

40 g 2-Keto-L-gulonsäure werden in einem Glaseinsatz vorgelegt und anschließend werden 180 g konz. Salzsäure hinzuaddiert. Nach Verschließen des Autoklaven wird Chlorwasserstoff so lange eingeleitet bis kein Druckabfall durch die Auflösung der HCI in der wäßrigen Phase mehr auftritt (ca. 20 min.). Der Autoklav wird anschließend innerhalb von 0,5 h auf 42 °C aufgewärmt und bei dieser Temperatur drei Stunden gehalten. Nach dem Abkühlen und Entspannen des Autoklaven wird die Reaktionsmischung am Rotationsverdampfer eingeengt. Gegen Ende fällt ein hellgraues kristallines Material an, welches zur Entfernung von Säurespuren nochmals in Butanol aufgenommen wird und anschließend wiederum zum Rückstand gebracht wird (Ausbeute: 93,3 % der Theorie).

### Beispiel 6

50 g 2-Keto-L-gulonsäure werden in einem Glaseinsatz vorgelegt und anschließend werden 150 g konz. Salzsäure hinzuaddiert. Nach Verschließen des Autoklaven wird Chlorwasserstoff so lange eingeleitet bis kein Druckabfall durch die Auflösung der HCl in der wäßrigen Phase mehr auftritt (ca. 20 min.; ca. 60 g HCI). Der Autoklav wird anschließend innerhalb von 0,5 h auf 42 °C aufgewärmt und bei dieser Temperatur drei Stunden gehalten. Nach dem Abkühlen und Entspannen des Autoklaven wird die Reaktionsmischung am Rotationsverdampfer eingeengt. Gegen Ende fällt ein hellgraues kristallines Material an, welches zur Entfernung von Säurespuren nochmals in Butanol aufgenommen wird und anschließend wiederum zum Rückstand gebracht wird (Ausbeute: 92,3 % der Theorie).

### Beispiel 7

3 g 2-Keto-L-gulonsäure werden in einem Glaseinsatz vorgelegt und anschließend werden 9 g konz. Salzsäure hinzuaddiert. Nach Verschließen des Autoklaven wird Chlorwasserstoff so lange eingeleitet bis kein Druckabfall durch die Auflösung der HCI in der wäßrigen Phase mehr auftritt (ca. 20 min.). Der Autoklav wird anschließend innerhalb von 0,5 h auf 45 °C aufgewärmt und bei dieser Temperatur zwei Stunden gehalten (Druck ca. 45 bar). Nach dem Abkühlen und Entspannen des Autoklaven wird die Reaktionsmischung am Rotationsverdampfer eingeengt. Gegen Ende fällt ein hellbraunes kristallines Material an, welches zur Entfernung von Säurespuren nochmals in Butanol aufgenommen wird und anschließend wiederum zum Rückstand gebracht wird (Ausbeute: 87,7 % der Theorie).

### Verfahrensvariante B

### Beispiel 8

50 g 2-Keto-L-gulonsäure werden in einem Glaseinsatz vorgelegt und anschließend werden 12 g Wasser hinzuaddiert. Nach Verschließen des Autoklaven werden 208 g Chlorwasserstoff in flüssiger Form eingeleitet. Der Autoklav wird anschließend innerhalb von 0,5 h auf 50 °C aufgewärmt und bei dieser Temperatur zwei Stunden gehalten. Nach der Aufwärmphase stellt sich ein Druck von ca. 80 bar ein. Nach dem Abkühlen und Entspannen des Autoklaven wird die Reaktionsmischung am Rotationsverdampfer eingeengt. Gegen Ende fällt ein grau-braunes kristallines Material an, welches zur Entfernung von Säurespuren nochmals in Butanol aufgenommen wird und anschließend wiederum zum Rückstand gebracht wird (Ausbeute: 75,3 % der Theorie).

### Beispiel 9

50 g 2-Keto-L-gulonsäure werden in einem Glaseinsatz vorgelegt und anschließend werden 12 g Wasser hinzuaddiert. Nach Verschließen des Autoklaven werden 110 g Chlorwasserstoff in flüssiger Form eingeleitet. Der Autoklav wird anschließend innerhalb von 0,5 h auf 45 °C aufgewärmt und bei dieser Temperatur drei Stunden gehalten. Nach der Aufwärmphase stellt sich ein Druck von ca. 76 bar ein. Nach dem Abkühlen und Entspannen des Autoklaven wird die Reaktionsmischung am Rotationsverdampfer eingeengt. Gegen Ende fällt ein grau-braunes kristallines Material an, welches zur Entfernung von Säurespuren nochmals in Butanol aufgenommen wird und anschließend wiederum zum Rückstand gebracht wird (Ausbeute: 83,3 % der Theorie).

### Vergleichsbeispiele

### Beispiel A

Vergleichsbeispiel mit 37 %iger Salzsäure bei 40 °C und Normaldruck: 100 g 2-Keto-gulonsäure werden in 300 g 37%ige Salzsäure eingetragen. Danach wird der Ansatz auf 40 °C aufgeheizt und bei dieser Temperatur ca. 4 h gehalten. Es wird eine schwarz-braun gefärbte Lösung enthalten. Die iodometrische Bestimmung des L-Ascorbinsäuregehaltes ergibt eine Ausbeute von 77,4 % der Theorie.

### Beispiel B

Vergleichsbeispiel mit 37 %iger Salzsäure bei 60 °C und Normaldruck: 100 g 2-Keto-L-gulonsäure werden in 300 g 37 %ige Salzsäure eingetragen. Danach wird der Ansatz auf 59-60 °C aufgeheizt und bei dieser Temperatur ca. 3 h gehalten. Es wird eine schwarz gefärbte Lösung enthalten. Die iodometrische Bestimmung des L-Ascorbinsäuregehaltes ergibt eine Ausbeute von 90,2 % der Theorie.

## Patentansprüche

1. Verfahren zur Herstellung von L-Ascorbinsäure aus 2-Keto-L-gulonsäure oder 2,3-4,6-Diaceton-2-keto-L-gulonsäure, **dadurch gekennzeichnet, daß** die Reaktion in Gegenwart von nur Wasser und Halogenwasserstoff durchgeführt wird, die Konzentration von Chlorwasserstoff in Wasser nach Zugabe aller Reaktionskomponenten von 40 bis 90 Gew.% und der Druck 10 - 100 bar beträgt.

2. Verfahren nach Anspruch 1, **dadurch gekennzeichnet, daß** die Reaktionstemperatur von 0 bis 60 °C beträgt.

3. Verfahren nach Anspruch 1 oder 2, **dadurch gekennzeichnet, daß** es in kontinuierlicher Fahrweise durchgeführt wird.

4. Verfahren nach einem oder mehreren der Ansprüche 1 bis 3, **dadurch gekennzeichnet, daß** der Halogenwasserstoff recycelt wird.

5. Verfahren nach einem oder mehreren der Ansprüche 1 bis 4, **dadurch gekennzeichnet, daß** die gewünschte Konzentration des Halogenwasserstoffs in Wasser durch Zugabe von gasförmigem oder flüssigem unverdünntem Halogenwasserstoff eingestellt wird.

## Claims

1. Process for the preparation of L-ascorbic acid from 2-keto-L-gulonic acid or 2,3-4,6-diacetone-2-keto-L-gulonic acid, **characterized in that** the reaction is carried out in the presence of just water and hydrogen halide, the concentration of hydrogen chloride in water following the addition of all the reaction components is from 40 to 90% by weight and the pressure is 10 - 100 bar.

2. Process according to Claim 1, **characterized in that** the reaction temperature is from 0 to 60°C.

3. Process according to Claim 1 or 2, **characterized in that** it is carried out in a continuous procedure.

4. Process according to one or more of Claims 1 to 3, **characterized in that** the hydrogen halide is recycled.

5. Process according to one or more of Claims 1 to 4, **characterized in that** the desired concentration of the hydrogen halide in water is set by addition of gaseous or liquid undiluted hydrogen halide.

## Revendications

1. Procédé de préparation d'acide L-ascorbique à partir d'acide 2-céto-L-gulonique ou d'acide 2,3-4,6-diacétone-2-céto-L-gulonique, **caractérisé en ce que** la réaction n'est réalisée qu'en présence d'eau et d'hydrogène halogéné, la concentration en acide chlorhydrique dans l'eau après ajout de tous les composants réactionnels s'élève de 40 à 90 % en poids et la pression de 10 à 100 bars.

2. Procédè selon la revendication 1, **caractérisé en ce que** la température réactionnelle s'èlève de 0 à 60°C.

3. Procéde selon la revendication 1 ou 2, **caractérisé en ce qu'**il est réalisé de façon continue.

4. Procédé selon l'une ou plusieurs des revendications 1 à 3, **caractérisé en ce que** l'hydrogène halogéné est recyclé.

5. Procédé selon l'une ou plusieurs des revendications 1 à 4, **caractérisé en ce que** la concentration souhaitée en hydrogène halogéné dans l'eau est ajustée par l'ajout d'hydrogène halogéné gazeux ou liquide non dilué.
